# EUROPÄISCHE PATENTSCHRIFT

(11) **EP 0 654 474 B1**
(45) Veröffentlichungstag und Bekanntmachung des Hinweises auf die Patenterteilung: **29.01.1997**
(21) Anmeldenummer: 94117962.4
(22) Anmeldetag: 15.11.1994
(51) Int. Cl.: C07D 487/04, G03G 9/135

(54) **Verdoppelte Benzimidazole und ihre Anwendung als Ladungsstabilisatoren**
Benzimidazole dimers and their use as charge stabilizers
Dimers de la benzimidazole et leur utilisation comme stabilisateurs de la charge

(30) Priorität: 24.11.1993 DE 4339959
(43) Veröffentlichungstag der Anmeldung: 24.05.1995
(73) Patentinhaber: BASF AKTIENGESELLSCHAFT, 67056 Ludwigshafen (DE)
(72) Erfinder: Schroeder, Dr. Gunter-Rudolf, D-69126 Heidelberg (DE); Mayer, Dr. Udo, D-67227 Frankenthal (DE); Beck, Dr. Karin Heidrun, D-67067 Ludwigshafen (DE); Dyllick-Brenzinger, Dr. Rainer, D-69469 Weinheim (DE)

(56) Entgegenhaltungen:
- EP-A- 0 575 805
- EP-A- 0 590 446
- DE-A- 2 733 468
- JP-A- 4 264 454
- US-A- 4 912 006

## Beschreibung

Die vorliegende Erfindung betrifft neue Benzimidazole der Formel I 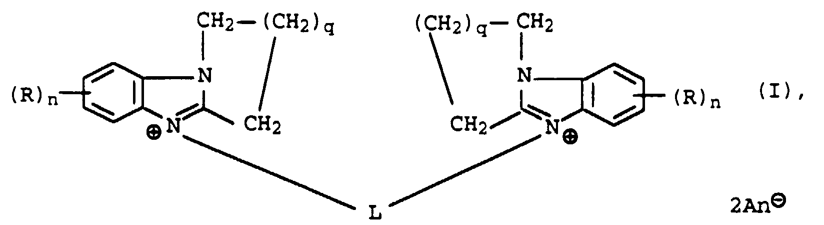 in der
- n und q: unabhängig voneinander jeweils 1 oder 2,
- die: Reste R unabhängig voneinander jeweils Wasserstoff, Chlor oder Methyl,
- L: C₂-C₁₀ Alkylen, C₄-C₁₀ Alkenylen oder ein Rest der Formel 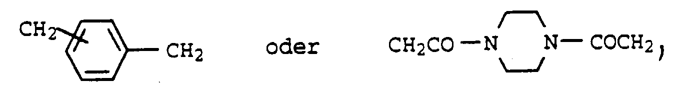
- An^{⊖}: das Äquivalent eines Anions bedeuten,
elektrostatische Toner, enthaltend die Benzimidazole als Ladungsstabilisatoren sowie die Verwendung der Benzimidazole als Ladungsstabilisatoren.

Aus der DE-A-2 733 468 und der US-A-4 912 006 sind bereits ähnliche Benzimidazolverbindungen bekannt. Es hat sich jedoch gezeigt, daß sie bei ihrer Anwendung als Ladungsstabilisatoren in elektrostatischen Tonern noch anwendungstechnische Mängel aufweisen. Weiterhin sind in der alteren Patentanmeldung EP-A-590 446 spezielle Phenylalkylbenzimidazole beschrieben.

Aufgabe der vorliegenden Erfindung war es nun, neue Benzimidazole bereitzustellen, die Über ein vorteilhaftes anwendungstechnisches Eigenschaftsprofil verfügen.

Demgemäß wurden die eingangs naher bezeichneten verdoppelten Benzimidazole der Formel I gefunden.

Alle in Formel I auftretenden Alkylen- oder Alkenylengruppen können sowohl geradkettig als auch verzweigt sein.

Geeignete Brückenglieder L sind. C₂-C₁₀-Alkylen, C₄-C₁₀-Alkenylen oder ein Rest der Formel 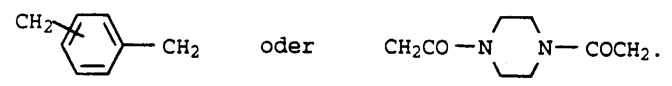 C₂-C₁₀-Alkylenreste sind z.B. (CH₂)₂, (CH₂)₃, (CH₂)₄, (CH₂)₅, (CH₂)₆, (CH₂)₇, (CH₂)₈, (CH₂)₉, (CH₂)₁₀, CH(CH₃)CH₂ oder CH(CH₃)CH(CH₃).
C₄-C₁₀-Alkenylenreste sind z.B. CH₂CH=CHCH₂, CH₂CH=CH(CH₂)₂ oder (CH₂)₂CH=CH(CH₂)₂.

Bevorzugte Brückenglieder L sind C₂-C₈-Alkylen, CH₂CH=CHCH₂, 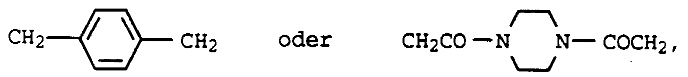 wobei C₂-C₈-Alkylen besonders hervorzuheben ist.

Bevorzugt sind Benzimidazole der Formel I, die einen symmetrischen Aufbau aufweisen.

Geeignete Anionen sind z.B. anorganische oder organische Anionen, beispielsweise Halogenide, wie Fluorid, Chlorid, Bromid oder Iodid, Hexafluorophosphat, Tetrafluoroborat, Formiat, Acetat, Propionat, Oxalat, Benzolsulfonat, Toluolsulfonat oder Tetraphenylboranat.

Besonders bevorzugt sind Benzimidazole der Formel I, in der
- q: jeweils 1,
- R: jeweils Wasserstoff und
- L: C₂-C₈-Alkylen bedeuten und
- n und An^{⊖}: jeweils die oben genannte Bedeutung besitzen.
Von besonderem Interesse sind Benzimidazole der Formel I, in der L C₂-C₅-Alkylen bedeutet.

Die erfindungsgemäßen Benzimidazole der Formel I können nach an sich bekannten Methoden, wie sie beispielsweise in der DE-A-2 733 468 beschrieben sind, erhalten werden.

So kann man ein verbrücktes Benzimidazol der Formel II 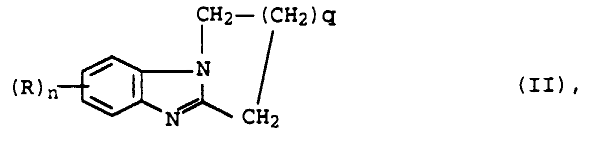 in der n, q und R jeweils die obengenannte Bedeutung besitzen, mit einer Verbindung der Formel III

X- L- X (III),

in der L die obengenannte Bedeutung besitzt und X für eine Austrittsgruppe, z.B. Chlor, Brom oder Iod, steht, umsetzen und gegebenenfalls anschließend mittels eines Salzes der Formel IV

M^{⊕} An^{⊖} (IV),

in der An^{⊖} die obengenannte Bedeutung besitzt und M^{⊕} für das Äquivalent eines Metallkations, z.B. Natrium oder Kalium, steht, ausfallen.

Die neuen Benzimidazole eignen sich vorteilhaft als Ladungsstabilisatoren in elektrostatischen Tonern.

Demgemäß betrifft die vorliegende Erfindung weiterhin elektrostatische Toner, enthaltend ein polymeres Bindemittel und als Ladungsstabilisator ein Benzimidazol der Formel I.

Der Anteil der Benzimidazole der Formel I im elektrostatischen Toner beträgt in der Regel 0,01 bis 10 Gew.-%, bezogen auf das Gewicht des Toners.

Die in den neuen elektrostatischen Tonern enthaltenen polymeren Bindemittel sind an sich bekannt. Sie sind in der Regel thermoplastisch und haben einen Erweichungspunkt von 40 bis 200°C, vorzugsweise 50 bis 130°C und insbesondere 65 bis 115°C. Beispiele für polymere Bindemittel sind Polystyrol, Copolymere von Styrol mit einem Acrylat oder Methacrylat, Copolymere von Styrol mit Butadien und/oder Acrylnitril, Polyacrylate, Polymethacrylate, Copolymere eines Acrylats oder Methacrylats mit Vinylchlorid oder Vinylacetat, Polyvinylchlorid, Copolymere von Vinylchlorid mit Vinylidenchlorid, Copolymere von Vinylchlorid mit Vinylacetat, Polyesterharze, Epoxyharze, Polyamide oder Polyurethane.

Zusätzlich zu den obengenannten Benzimidazolen I und den polymeren Bindemitteln können die erfindungsgemäßen Toner in bekannten Mengen Farbmittel, magnetisch anziehbares Material, Wachse und Fließmittel enthalten.

Die Farbmittel können organische Farbstoffe oder Pigmente, wie Nigrosin, Anilinblau, 2,9-Dimethylchinacridon, C.I. Disperse Red 15 (C.I. 6010), C.I. Solvent Red 19 (C.I. 26 050), C.I. Pigment Blue 15 (C.I. 74 160), C.I. Pigment Blue 22 (C.I. 69 810) oder C.I. Solvent Yellow 16 (C.I. 12 700) oder anorganische Pigmente, wie Ruß, Rotblei, gelbes Bleioxid oder Chromgelb, sein. Allgemein überschreitet die Menge des im Toner vorhandenen Farbmittels nicht 15 Gew.-%, bezogen auf das Gewicht des Toners.

Das magnetisch anziehbare Material kann beispielsweise Eisen, Nickel, Chromoxid, Eisenoxid oder ein Ferrit der Formel MeFe₂O₄, worin Me ein zweiwertiges Metall, z.B. Eisen, Kobalt, Zink, Nickel oder Mangan, darstellt, sein.

Die Herstellung der erfindungsgemäßen Toner erfolgt nach üblichen Verfahren, z.B. durch Vermischen der Bestandteile in einem Kneter und anschließendes Pulverisieren oder durch Schmelzen des polymeren Bindemittels oder eines Gemisches der polymeren Bindemittel, anschließende feine Zerteilung eines oder mehrerer Benzimidazole I, sowie der anderen Zusätze, falls verwendet, in dem geschmolzenen Harz unter Anwendung der für diesen Zweck bekannten Misch- und Knetmaschinen, anschließende Abkühlung der Schmelze zu einer festen Masse und schließlich Vermahlen der festen Masse zu Teilchen der gewünschten Teilchengröße (in der Regel 0,1 bis 50 µm). Es ist auch möglich, das polymere Bindemittel und den Ladungsstabilisator in einem gemeinsamen Lösungsmittel zu suspendieren und die anderen Zusätze in die Suspension zu geben. Die Suspension kann so als Flüssigtoner verwendet werden.

Man kann die Flüssigkeit aber auch in an sich bekannter Weise sprühtrocknen, die Lösungsmittel abdampfen oder die Flüssigkeit gefriertrocknen und den festen Rückstand zu Teilchen der gewünschten Teilchengröße vermahlen.

Es ist weiterhin möglich, die als Ladungstabilisatoren verwendeten neuen Benzimidazole nicht zu lösen, sondern fein in der Lösung des polymeren Bindemittels zu dispergieren. Die so erhaltene Tonerzubereitung kann dann, beispielsweise gemäß der US-A-4 265 990, in einem xerographischen Bildaufzeichnungssystem verwendet werden.

Die obengenannten Benzimidazole der Formel I sind vorteilhafte Ladungsstabilisatoren. Sie zeichnen sich besonders dadurch aus, daß sie bei Zusatz zu einer Tonerpräparation dieser ein günstiges elektrostatisches Aufladungsprofil verleihen, d.h. die Toner lassen sich schnell und hoch aufladen. Die erfindungsgemäßen Ladungsstabilisatoren bewirken weiterhin, daß die Ladung auf einem hohen Niveau konstant gehalten wird.

Die folgenden Beispiele sollen die Erfindung näher erläutern.

### A) Herstellung der Benzimidazole

### Beispiel H1

79,0 g (0,5 mol) Pyrrolidino[1,2-a]benzimidazol wurden in 90 ml Ethylenglykolmonobutylether gelöst und auf 120°C erhitzt. Man ließ während 1 h 31,75 g (0,25 mol) 1,4-Dichlorbutan zutropfen. Dann erhöhte man die Temperatur auf 140°C und ließ 2 h nachrühren. Nach dem Abkühlen auf 90°C ließ man 300 ml heißes Wasser zulaufen und rührte 2 h nach. Unter Rühren wurden dann 60,4 g (0,55 mol) Natriumtetrafluoroborat zugegeben. Der kristalline Niederschlag wurde nach 1 h bei 50°C abgesaugt und mit Wasser gewaschen. Das wasserfeuchte Nutschgut wurde unter vermindertem Druck bei 60°C getrocknet. Man erhielt auf diese Weise 86 g eines grauweißen Produkts der Formel

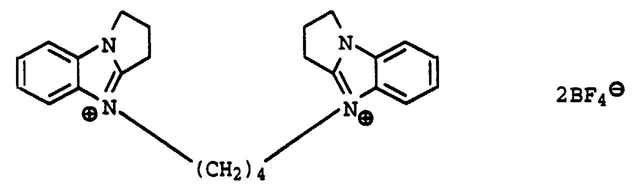

In analoger Weise werden die folgenden Benzimidazole erhalten.

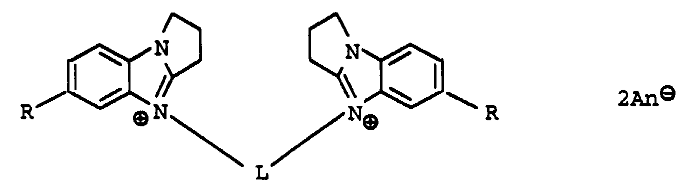

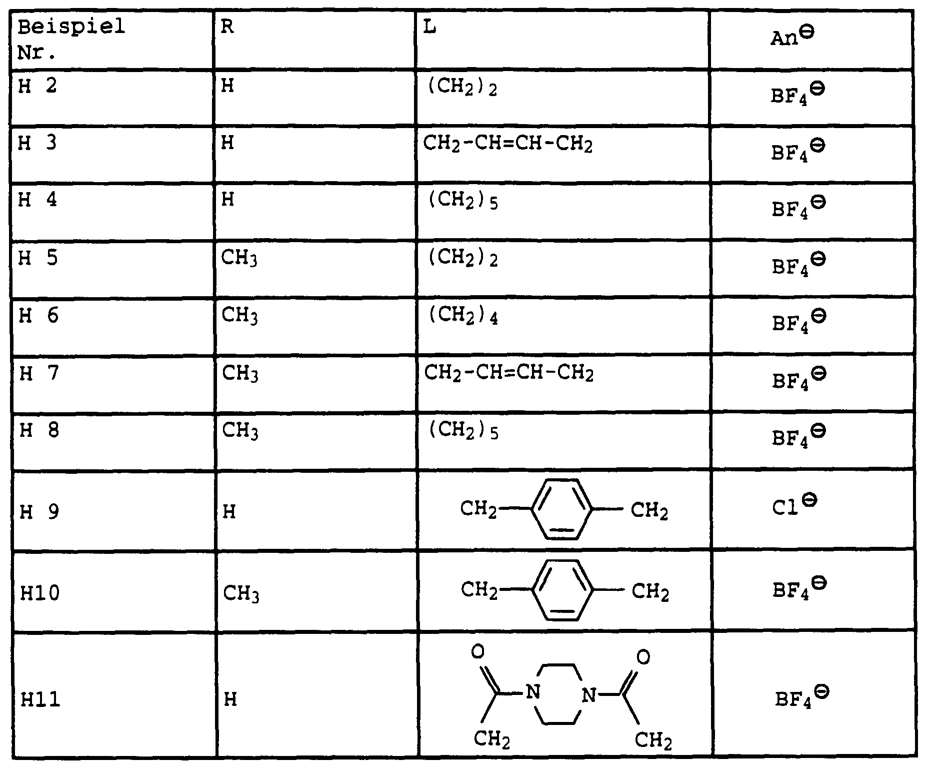

### B) Anwendung

Die Anwendungsbeispiele wurden mit farbmittelfreien Tonermodellen, bestehend aus Harz und den erfindungsgemäßen Ladungsstabilisatoren, durchgeführt.

### I. Herstellung der Toner

In eine Lösung von 10 g eines linearen, nicht vernetzten Polyesterharzes in 100 ml Xylol wurden bei Raumtemperatur 0,2 g Benzimidazol eingetragen und anschließend gefriergetrocknet. Das resultierende Produkt wurde dann gemahlen (mittlere Teilchengröße: 50 µm).

### II. Herstellung der Developer und Prüfung

Zur Herstellung eines Developers wurden 99 Gew.-% eines Stahlcarriers, der eine mittlere Teilchengröße von 100 µm aufwies, mit 1 Gew.-% des Toners genau eingewogen und über einen unten näher bestimmten Zeitraum auf einem Rollenbock aktiviert. Danach wurde die elektrostatische Aufladung des Developers bestimmt. Etwa 5 g des aktivierten Developers wurden in einem handelsüblichen q/m Meter (Firma Epping GmbH, Neufahrn) in eine hard-blow-off-Zelle, die mit einem Elektrometer elektrisch verbunden war, eingefüllt. Die Maschenweiten der in der Meßzelle eingesetzten Siebe betrug 63 µm.

Damit war gewährleistet, daß der Toner möglichst vollständig ausgeblasen wurde, der Carrier aber in der Meßzelle verblieb. Durch einen kräftigen Luftstrom (ca. 4 000 cm³/min) und gleichzeitigem Absaugen wurde der Toner nahezu vollständig von den Carrierteilchen entfernt, wobei letztere in der Meßzelle verblieben. Die Aufladung des Carriers wurde am Elektrometer registriert. Sie entsprach dem Betrag der Aufladung der Tonerteilchen, nur mit umgekehrten Vorzeichen. Zur Berechnung des q/m-Wertes wurde deshalb der Betrag von q mit den umgekehrten Vorzeichen verwendet. Durch Zurückwiegen der Meßzelle wurde die Masse an ausgeblasenem Toner bestimmt und daraus die elektrostatische Aufladung q/m berechnet.

Die an den Tonern bestimmte Aufladung ist in der folgenden Tabelle zusammengefaßt.

**Tabelle**

| Beispiel Nr. | Verbindung aus Beispiel | Aufladung nach einer Aktivierung von | | | |
|---|---|---|---|---|---|
| | | 10 min | 30 min | 60 min | 120 min |
| | | [µC/g] | | | |
| A 1 | H 1 | 20,8 | 17,9 | 17,9 | 17,3 |
| A 2 | H 2 | 18,8 | 18,8 | 18,2 | 18,8 |
| A 3 | H 3 | 19,2 | 19,7 | 19,6 | 19,9 |
| A 4 | H 4 | 17,9 | 17,5 | 22,1 | 18,7 |
| A 5 | H 5 | 19,9 | 21,4 | 19,7 | 18,7 |
| A 6 | H 6 | 20,9 | 20,1 | 20,1 | 21,6 |
| A 7 | H 7 | 19,4 | 19,2 | 18,1 | 17,1 |
| A 8 | H 8 | 16,9 | 19,3 | 19,4 | 17,7 |
| A 9 | H 9 | | 13,1 | | |
| A10 | H10 | 15,2 | 14,7 | 15,5 | 14,8 |
| A11 | H11 | | 8,4 | | |

## Patentansprüche (Patentansprüche für folgende(n) Vertragsstaat(en): BE, CH, DE, FR, GB, IE, IT, LI)

1. Benzimidazole der Formel I 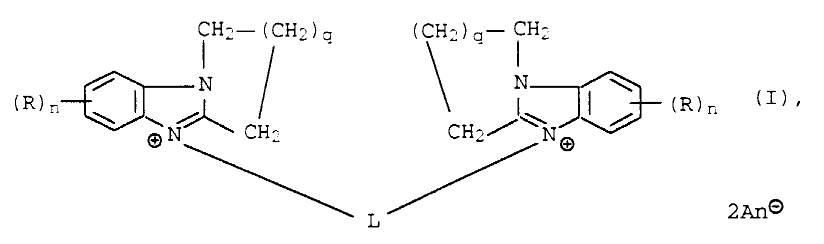 in der
n und q unabhängig voneinander jeweils 1 oder 2,
die Reste R unabhängig voneinander jeweils Wasserstoff, Chlor oder Methyl,
L C₂-C₁₀-Alkylen, C₄-C₁₀-Alkenylen oder ein Rest der Formel 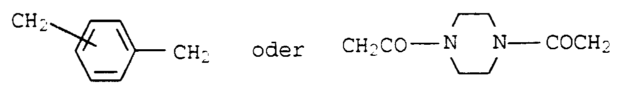 und
An^{⊖} das Äquivalent eines Anions bedeuten.

2. q jeweils 1,
R jeweils Wasserstoff und
L C₂-C₈-Alkylen bedeuten.

3. Elektrostatische Toner, enthaltend ein polymeres Bindemittel und als Ladungsstabilisator ein Benzimidazol gemäß Anspruch 1.

4. Verwendung der Benzimidazole gemäß Anspruch 1 als Ladungsstabilisatoren in elektrostatischen Tonern.

## Patentansprüche (Patentansprüche für folgende(n) Vertragsstaat(en): ES)

1. Verfahren zur Herstellung von Benzimidazolen der Formel I 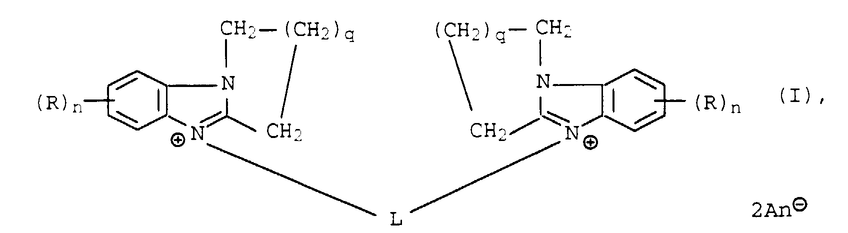 in der
n und q unabhängig voneinander jeweils 1 oder 2,
die Reste R unabhängig voneinander jeweils Wasserstoff, Chlor oder Methyl,
L C₂-C₁₀-Alkylen, C₄-C₁₀-Alkenylen oder ein Rest der Formel 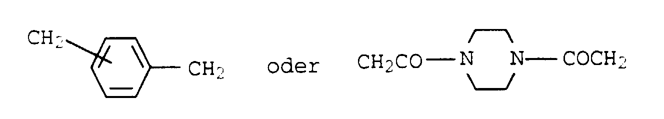 und
An^{⊖} das Äquivalent eines Anions bedeuten,
dadurch gekennzeichnet, daß man ein verbrücktes Benzimidazol der Formel II 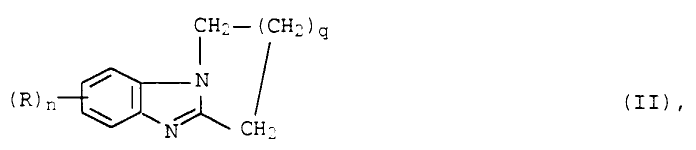 in der n, q und R jeweils die obengenannte Bedeutung besitzen, mit einer Verbindung der Formel III
X-L-X (III),
in der L die obengenannte Bedeutung besitzt und X für eine Austrittsgruppe steht, umsetzt und gegebenenfalls anschließend mittels eines Salzes der Formel IV
M^{⊕} An^{⊖} (IV),
in der An^{⊖} die obengenannte Bedeutung besitzt und M^{⊕} für das Äquivalent eines Metallkations steht, ausfällt.

2. Verfahren nach Anspruch 1, dadurch gekennzeichnet, daß
q jeweils 1,
R jeweils Wasserstoff und
L C₂-C₈-Alkylen bedeuten.

3. Elektrostatische Toner, enthaltend ein polymeres Bindemittel und als Ladungsstabilisator ein Benzimidazol gemäß Anspruch 1.

4. Verwendung der Benzimidazole gemäß Anspruch 1 als Ladungsstabilisatoren in elektrostatischen Tonern.

## Claims (Claims for the following Contracting State(s): BE, CH, DE, FR, GB, IE, IT, LI)

1. A benzimidazole of the formula I 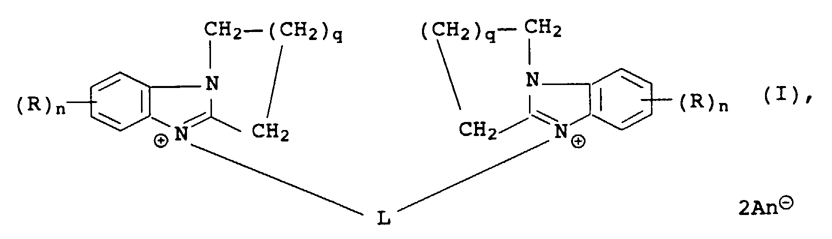 where
n and q independently of one another are each 1 or 2,
the radicals R independently of one another are each hydrogen, chlorine or methyl,
L is C₂-C₁₀-alkylene, C₄-C₁₀-alkylene or a radical of the formula 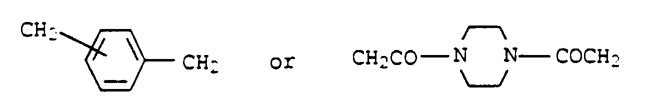 and
An^{⊖} is one equivalent of an anion.

2. A benzimidazole as claimed in claim 1, wherein
q is 1,
the radicals R are each hydrogen and
L is C₂-C₈-alkylene.

3. An electrostatic toner containing a polymeric binder and, as a charge stabilizer, a benzimidazole as claimed in claim 1.

4. Use of a benzimidazole as claimed in claim 1 as a charge stabilizer in electrostatic toners.

## Claims (Claims for the following Contracting State(s): ES)

1. A process for the preparation of a benzimidazole of the formula I 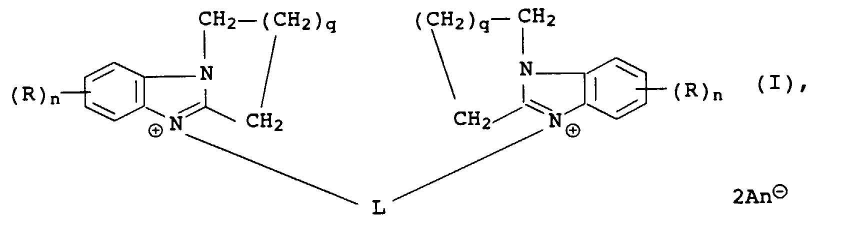 where
n and q independently of one another are each 1 or 2,
the radicals R independently of one another are each hydrogen, chlorine or methyl,
L C₂-C₁₀-alkylene, C₄-C₁₀-alkenylene or a radical of the formula 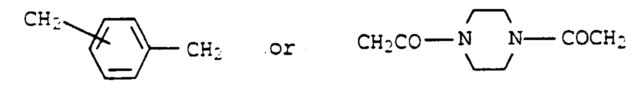 and
An^{⊖} is one equivalent of an anion,
wherein a bridged benzimidazole of the formula II 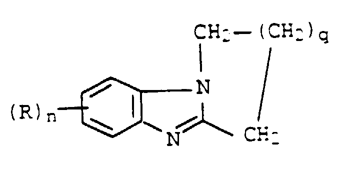 where n, q and r each have the abovementioned meanings, is reacted with a compound of the formula III
X-L-X (III),
where L has the abovementioned meanings and X is a leaving group, and, if required, the product is then precipitated by means of a salt of the formula IV
M^{⊕} An^{⊖} (IV),
where An^{⊖} has the abovementioned meaning and M^{⊕} is one equivalent of a metal cation,

2. A process as claimed in claim 1, wherein
q is 1,
the radicals R are each hydrogen and
L is C₂-C₈-alkylene.

3. An electrostatic toner containing a polymeric binder and, as a charge stabilizer, a benzimidazole as claimed in claim 1.

4. Use of a benzimidazole as claimed in claim 1 as a charge stabilizer in electrostatic toners.

## Revendications (Revendications pour l'(les) Etat(s) contractant(s) suivant(s): BE, CH, DE, FR, GB, IE, IT, LI)

1. Benzimidazole de la formule I 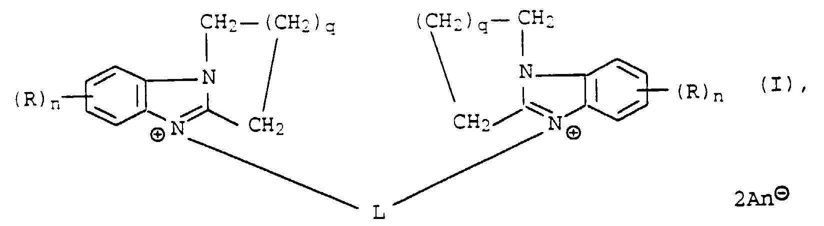 dans laquelle
n et q représentent, chacun indépendamment l'un de l'autre, 1 ou 2,
les symboles R représentent chacun indépendamment l'un de l'autre, un atome d'hydrogène, un atome de chlore ou le radical méthyle,
L représente un groupe alkylène en C₂ à C₁₀, alcénylène en C₄ à C₁₀, ou un reste de la formule 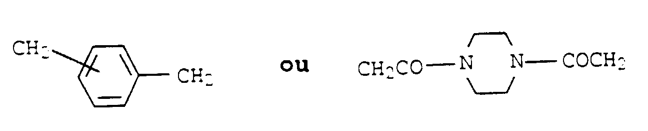 et
An^{⊖} représente l'équivalent d'un anion.

2. Benzimidazole suivant la revendication 1, caractérisé en ce que
q est à chaque fois 1,
R est à chaque fois l'hydrogène et,
L est à chaque fois un radical alkylène en C₂ à C₈.

3. Encres électrostatiques, qui contiennent un liant polymérique et, à titre de stabilisateur de charges, un benzimidazole suivant la revendication 1.

4. Utilisation des benzimidazoles suivant la revendication 1, à titre de stabilisateurs de charges dans des encres électrostatiques.

## Revendications (Revendications pour l'(les) Etat(s) contractant(s) suivant(s): ES)

1. Procédé de préparation de benzimidazole de la formule I 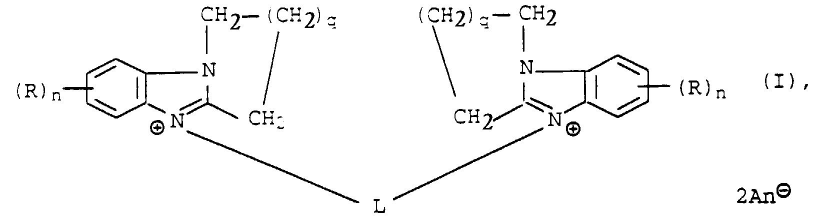
n et q représentent, chacun indépendamment l'un de l'autre, 1 ou 2,
les symboles R représentent chacun indépendamment l'un de l'autre, un atome d'hydrogène, un atome de chlore ou le radical méthyle,
L représente un groupe alkylène en C₂ à C₁₀, alcénylène en C₄ à C₁₀, ou un reste de la formule 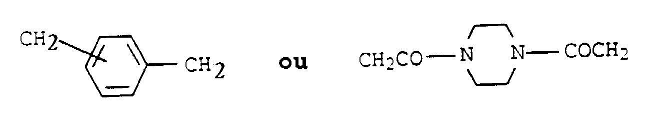
et An^{⊖} représente l'équivalent d'un anion
caractérisé en ce que l'on fait réagir un benzimidazole ponté de la formule II 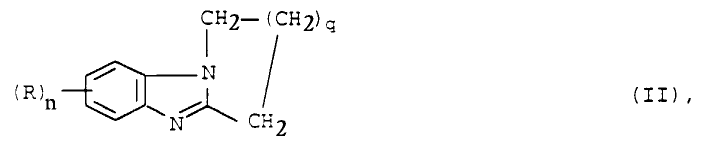 dans laquelle,
n, q, et R possèdent les significations qui leur ont été précédemment attribuées, avec un composé de la formule III
X-L-X (III),
dans laquelle L possède les significations qui lui ont été précédemment attribuées et X représente un groupe sortant et on précipite ensuite éventuellement un cation de métal à l'aide d'un sel de la formule IV
M^{⊕}An^{⊖}
dans laquelle An^{⊖} possède les significations qui lui ont été précédemment attribuées et M^{⊕} représente l'équivalent d'un cation de métal.

2. Procédé suivant la revendication 1, caractérisé en ce que
q est à chaque fois 1,
R est à chaque fois l'hydrogène et,
L est à chaque fois un radical alkylène en C₂ à C₈.

3. Encres électrostatiques, qui contiennent un liant polymérique et, à titre de stabilisateur de charges, un benzimidazole suivant la revendication 1.

4. Utilisation des benzimidazoles suivant la revendication 1, à titre de stabilisateurs de charges dans des encres électrostatiques.
